(19) Europäisches Patentamt
European Patent Office
Office européen des brevets

(11) Veröffentlichungsnummer : **0 470 099 B1**

# (12) EUROPÄISCHE PATENTSCHRIFT

(45) Veröffentlichungstag der Patentschrift :
**03.11.93 Patentblatt 93/44**

(51) Int. Cl.$^5$ : **A61K 7/13,** C07D 319/18, C07D 321/00

(21) Anmeldenummer : **90906152.5**

(22) Anmeldetag : **17.04.90**

(86) Internationale Anmeldenummer :
**PCT/EP90/00596**

(87) Internationale Veröffentlichungsnummer :
**WO 90/12562 01.11.90 Gazette 90/25**

## (54) HAARFÄRBEMITTEL.

(30) Priorität : **24.04.89 DE 3913477**

(43) Veröffentlichungstag der Anmeldung :
**12.02.92 Patentblatt 92/07**

(45) Bekanntmachung des Hinweises auf die Patenterteilung :
**03.11.93 Patentblatt 93/44**

(84) Benannte Vertragsstaaten :
**AT BE CH DE FR GB IT LI LU NL SE**

(56) Entgegenhaltungen :
**EP-A- 0 004 366**
**GB-A- 1 005 899**
**Melliand Textilberichte, volume 40, No. 4, April 1959, H.C.A. Van Beek et al.: "Einige Azofarbstoffe, von Benzo -1:4 - Dioxan abgeleitet", pages 417-419**

(73) Patentinhaber : **Henkel Kommanditgesellschaft auf Aktien**
**D-40191 Düsseldorf (DE)**

(72) Erfinder : **ROSE, David**
**Am Eichelkamp 223**
**D-4010 Hilden (DE)**
Erfinder : **LIESKE, Edgar**
**Hunsrückenstrasse 40**
**D-4000 Düsseldorf (DE)**
Erfinder : **HÖFFKES, Horst**
**Carlo-Schmid-Strasse 113**
**D-4000 Düsseldorf-Hellerhof (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 470 099 B1

Jouve, 18, rue Saint-Denis, 75001 PARIS

## Beschreibung

Die Erfindung betrifft neue Haarfärbemittel auf der Basis von Oxidationsfarbstoffen, die als Oxidationsfarbstoffvorprodukte 2,3-Alkylendioxy-p-phenylendiamine enthalten.

Für das Färben von Haaren spielen die sogenannten Oxidationshaarfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf menschlichem Haar besitzen, wobei keine merklichen Unterschiede zwischen strapaziertem und frisch nachgewachsenem Haar bestehen dürfen. Sie sollen beständig sein gegen Licht, Wärme und den Einfluß chemischer Reduktionsmittel, z. B. gegen Dauerwellflüssigkeiten. Schließlich sollen sie die Kopfhaut nicht zu sehr anfärben, und vor allem sollen sie in toxikologischer und dermatologischer Hinsicht unbedenklich sein.

Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate und 4-Aminopyrazolonderivate eingesetzt. Als sogenannte Kupplersubstanzen werden m-Phenylendiaminderivate, Naphthole, Resorcinderivate und Pyrazolone verwendet.

Besonders wichtig sind die Intensität der bei der oxidativen Kupplung gebildeten Farbnuancen und die Echtheitseigenschaften, insbesondere die Lichtechtheit.

P-Phenylendiamin ist seit langem als Entwicklerkomponente für Oxidationshaarfärbemittel bekannt und es entwickelt auch mit vielen Kupplern intensive und stabile Färbungen. Derivate des p-Phenylendiamins verhalten sich jedoch in dieser Hinsicht meist weniger günstig als der Grundkörper. Es war daher überraschend, daß die neu aufgefundenen Entwicklerkomponenten mit sehr vielen Kupplern noch intensivere Oxidationsfärbungen mit noch besseren Echtheitseigenschaften ausbilden.

Gegenstand der Erfindung sind Haarfärbemittel mit einem Gehalt an Oxidationsfarbstoffvorprodukten in einem kosmetischen Träger, dadurch gekennzeichnet, daß als Oxidationsfarbstoffvorprodukt wenigstens eine Verbindung der Formel I

(I)

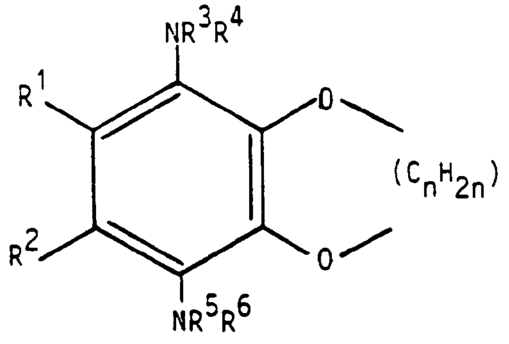

...

in der $R^1$ und $R^2$ unabhängig voneinander Wasserstoff, Halogenatome oder Alkylgruppen mit 1 - 4 C-Atomen und $R^3$, $R^4$, $R^5$ und $R^6$ unabhängig voneinander Wasserstoff, Alkylgruppen mit 1 - 4 C-Atomen oder Hydroxyalkylgruppen mit 2 - 4 C-Atomen sind und n eine ganze Zahl von 2 - 4 ist, oder deren Salz, als Entwicklerkomponente enthalten ist.

Die Verbindungen der Formel (I) sind zum Teil literaturbekannt; so ist z. B. das 5,8-Diamino-benzo-1,4-dioxan (2,3-Ethylendioxy-p-phenylendiamin) aus J. Chem. Soc. (London), 1954, Seite 20 - 21, bekannt. Andere Verbindungen der Formel I lassen sich nach literaturbekannten Verfahren herstellen. So läßt sich z. B. das 6,9-Diamino-3,4-dihydro-2H-1,5-benzodioxepin (2,3-Propylendioxy-p-phenylendiamin) aus 7,8-Dibrom-3,4-dihydro.2H-1,5-benzodioxepin, dessen Herstellung in J. Chem. Soc. (B), 1971, Seite 1239 - 1240 beschrieben ist, durch Nitrierung und katalytische Reduktion mit Wasserstoff erhalten (vgl. Beispiel 1.1).

Die Synthese der Verbindung der Formel I, in welcher $R^1$ und $R^2$ Chlor, $R^3$, $R^4$, $R^5$ und $R^6$ Wasserstoff und n = 2 ist, ist ebenfalls in J. Chem. Soc. (London), 1954, Seite 21 (Verb. XIV), beschrieben.

Wegen der besonders leichten Zugänglichkeit und der guten Anwendungseigenschaften sind die Verbindungen der Formel I, in welchen $R^1$ und $R^2$ Wasserstoff, Brom oder Chlor, $R^3$, $R^4$, $R^5$ und $R^6$ Wasserstoff und

$-C_nH_{2n}-$ eine Ethylen- oder 1,3-Propylengruppe ist, und deren wasserlösliche Salze bevorzugt. 5,8-Diamino-benzo-1,4-dioxan ist eine ganz besonders bevorzugte Verbindung der Formel I.

Die Verbindungen der Formel (I) oder deren Salze eignen sich als Oxidationsfarbstoffvorprodukte vom Entwickler-Typ; sie vermögen daher unter Einwirkung von Oxidationsmitteln Farbstoffe auszubilden. Besonders intensive und brillante Farbstoffe werden jedoch durch oxidative Kupplung in Gegenwart von sogenannten Kupplerkomponenten gebildet. Als Kuppler eignen sich alle Verbindungen, die als Kuppler für z. B. p-Phenylendiamin bekannt sind; dies sind in erster Linie aromatische Amine und Phenole, die in m-Position eine weitere Amino- oder Hydroxylgruppe tragen sowie Naphthole, Dihydroxynaphthaline, Aminopyridine, Aminopyrazolone und deren Derivate. Besonders lichtechte Haaranfärbungen werden erhalten, wenn die erfindungsgemäßen Haarfärbemittel Kupplerkomponenten enthalten, die aus den Substanzklassen der Aminophenole, Resorcine, Naphthole oder Dihydroxynaphthaline ausgewählt sind.

Besonders bevorzugte Kupplerkomponenten aus der Klasse der Aminophenole sind das 5-Amino-4-chlor-2-methylphenol, das 3-Amino-2-chlor-6-methylphenol und das 2,4-Dichlor-3-aminophenol.

Innerhalb der Klasse der Resorcine ist das Resorcin selbst besonders bevorzugt. Das Naphthol-1 stellt eine besonders geeignete Kupplerkomponente aus der Gruppe der Naphthole dar. Unter den Dihydroxynaphthalinen ist das 1,7-Dihydroxynaphthalin besonders bevorzugt.

Zur Modifikation der Farbnuance sind nicht nur die genannten Kupplerverbindungen geeignet, sondern auch weitere, literaturbekannte Entwicklerverbindungen und direktziehende Haarfarbstoffe.

Geeignete Entwicklerkomponenten sind vor allem aromatische Amine, die in ortho- oder para-Position eine zweite, substituierte oder freie Aminogruppe, eine Hydroxylgruppe, eine Oxyalkyl- oder eine Thioethergruppe tragen sowie Diaminopyridine, 4-Aminopyrazolone und 2,4,5,6-Tetraaminopyridine. Als direktziehende Haarfarbstoffe sind z. B. Nitrophenylendiaminderivate, Anthrachinonfarbstoffe oder Indophenole geeignet.

Die 2,3-Alkylendioxy-p-phenylendiamine der Formel (I) können entweder als solche oder in Form ihrer wasserlöslichen Salze mit anorganischen oder organischen Säuren, z. B. als Hydrochloride, Sulfate, Phosphate, Acetate, Propionate, Lactate oder Citrate isoliert und in Haarfärbemitteln eingesetzt werden.

In den erfindungsgemäßen Haarfärbemitteln werden die Entwicklersubstanzen und die Kupplersubstanzen im allgemeinen in äquimolaren Mengen eingesetzt, ein gewisser Überschuß einzelner Oxidationsfarbstoffvorprodukte ist jedoch nicht nachteilig, so daß Entwicklersubstanzen und Kupplersubstanzen in einem Molverhältnis von 1 : 0,5 bis 1 : 2 eingesetzt werden können. Die erfindungsgemäß einzusetzenden 2,3-Alkylendioxy-p-phenylendiamine der Formel (I) oder deren Salze können in einer Menge von 0,05 bis 10 Millimol pro 100 g des Haarfärbemittels verwendet werden. Es ist dabei nicht erforderlich, daß die Verbindungen der Formel (I) einheitliche Verbindungen sind. Vielmehr können auch Gemische der Verbindungen zum Einsatz kommen.

Zur Herstellung der erfindungsgemäßen Haarfärbemittel werden die Oxidationsfarbstoffvorprodukte und gegebenenfalls direktziehende Farbstoffe in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt.

Eine bevorzugte Ausführungsform der erfindungsgemäßen Haarfärbemittel sind Cremehaarfärbemittel in Form einer Öl-in-Wasser-Emulsion mit einem Gehalt von

| | |
|---|---|
| 1 bis 10 Millimol | pro 100 g an Entwicklerkomponenten, |
| 1 bis 10 Millimol | pro 100 g an Kupplerkomponenten, |
| 1 bis 10 Gew.-% | eines Alkyl($C_{10}$-$C_{18}$)-sulfat- oder Alkyl ($C_{10}$-$C_{16}$)-ethersulfattensids, |
| 5 bis 20 Gew.-% | eines Fettalkohols oder Fettalkoholgemisches mit 12 bis 18 C-Atomen, |
| 0,1 bis 2 Gew.-% | eines Oxidationsinhibitors, bevorzugt aus der Gruppe Alkalisulfit, Alkaliascorbat oder Alkali-dithionit |

sowie Ammoniak in einer Menge, um den pH-Wert der Emulsion auf einen Wert zwischen 8 und 10 einzustellen.

Das genannte Alkylsulfat- oder Alkylethersulfattensid kann als Alkali-, Ammonium- oder Alkanolammoniumsalz mit 2 oder 3 C-Atomen in der Alkanolgruppe vorliegen, z. B. als Natrium-, Triethanolammonium- oder Isopropanolammoniumsalz. Als Alkyl-$C_{10}$-$C_{16}$-ethersul-fattensid kann ein Schwefelsäuremonoestersalz eines Anlagerungsproduktes von 1 bis 10 Mol Ethylenoxid an einen $C_{10}$-$C_{16}$-Fettalkohol eingesetzt werden.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Die Anwendung der erfindungsgemäßen Haarfärbemittel kann, unabhängig von der Art der kosmetischen Zubereitung, z. B. als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

## Beispiele

1. Herstellungsbeispiel

1.1 Herstellung von 6,9-Diamino-3,4-dihydro-2H-1,5-benzodioxepin-dihydrochlorid

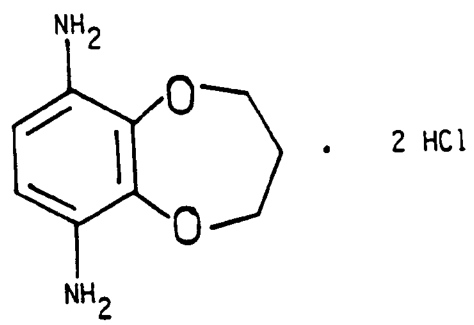

### 1. Stufe

In einem 250 ml Rührkolben wurden 70 ml Salpetersäure (D = 1,52) gegeben und bei einer Temperatur von 0 °C (± 5 °C) wurden 13 g 7,8-Dibrom-3,4-dihydro-2H-1,5-benzodioxepin (nach J. Chem. Soc. (8) 1971, 1235) portionsweise zugegeben. Die Mischung wurde noch 1,5 Stunden gerührt und dann auf Eis gegossen. Es bildete sich ein Niederschlag, der abfiltriert, bis zur Neutralität mit Wasser gewaschen und dann im Vakuum getrocknet wurde. Die Ausbeute an 7,8-Dibrom-6,9-dinitro-3,4-dihydro-2H-1,5-benzodioxepin betrug 15,5 g (92,7 % d. Th.). Das Produkt hatte einen Schmelzpunkt von 283 °C.

### 2. Stufe

Eine Suspension von 8,0 g 7,8-Dibrom-6,9-dinitro-3,4-dihydro-2H-1,5-benzodioxepin in 100 ml Ethanol wurde nach Zugabe von 50 ml Wasser und 1,76 g Natriumhydroxid sowie von 0,6 g Palladium auf Aktivkohle (5 % Pd) als Katalysator in Gegenwart von Wasserstoffgas reduziert, wobei gleichzeitig die Bromatome gegen Wasserstoff ausgetauscht wurden.

Nach ca. 7 Stunden war die berechnete Menge Wasserstoff aufgenommen. Der Ansatz wurde dann mit 3900 ml Wasser verdünnt und der Katalysator durch Filtration abgetrennt. Nach Zugabe von 50 ml 50%iger wäßriger Natriumhydroxidlösung wurde das Filtrat zur Trockene eingeengt. Der Rückstand wurde mit Toluol extrahiert. Die Lösung in Toluol wurde mit wäßriger Salzsäure extrahiert und die salzsauren, wäßrigen Extrakte zur Trockene eingeengt.
Ausbeute: 1,75 g (35 % d. Th.)
Schmelzpunkt: 230 - 250 °C (unter Zersetzung).

2. Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12-14}$ | 10,0 g |
| Fettalkohol $C_{12-14}$ + EO-sulfat, | |
| Na-Salz, 28%ig | 25,0 g |
| Wasser | 60,0 g |
| Entwicklerkomponente | 7,5 mMol |
| $Na_2SO_3$(Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als erfindungsgemäße Entwicklerkomponenten wurden eingesetzt:

E1: 5,8-Diaminobenzo-1,4-dioxan-dihydrochlorid

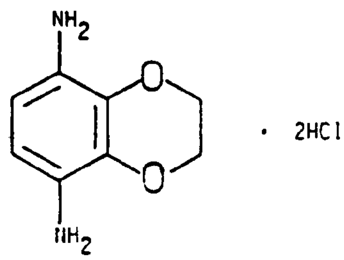

E2: 6,9-Diamino-3,4-dihydro-2H-1,5-benzodioxepindihydrochlorid (gemäß Beispiel 1)

Als Kuppler wurden die aus Tabelle I ersichtlichen Verbindungen eingesetzt. Dabei wurden die aus Tabelle II ersichtlichen Haaranfärbungen erhalten.

Tabelle I: Kupplerkomponenten

K 1: Resorcin
K 2: 5-Amino-2-methylphenol
K 3: 2-Chlor-3-amino-6-methylphenol
K 4: 1-Naphthol
K 5: 5-Amino-2-methyl-4-chlorphenol
K 6: 2,7-Dihydroxynaphthalin
K 7: 1,3-Bis-(2,4-diaminophenoxy)-propan
K 8: 1-Phenyl-3-methyl-pyrazolon-5
K 9: 2,6-Dihydroxy-3,4-dimethylpyridin
K10: m-Aminophenol
K11: 4-Chlorresorcin
K12: 1,5-Dihydroxynaphthalin
K13: 2,4-Dichlor-3-aminophenol
K14: 1,7-Dihydroxynaphthalin
K15: 1-Phenyl-3-aminopyrazolon-5

Tabelle II

| Beispiel Nr. | Entwickler | Kuppler | erhaltener Farbton |
|---|---|---|---|
| 2.1 | E1 | K1 | dunkelbraun |
| 2.2 | E2 | K1 | olivbraun |
| 2.3 | E1 | K2 | dunkelviolett |
| 2.4 | E2 | K2 | magenta |
| 2.5 | E1 | K3 | dunkelviolett |
| 2.6 | E2 | K3 | mattviolett |
| 2.7 | E1 | K4 | blauschwarz |
| 2.8 | E1 | K5 | dunkelviolett |
| 2.9 | E1 | K6 | braungrau |
| 2.10 | E1 | K7 | blauschwarz |
| 2.11 | E1 | K8 | violettbraun |
| 2.12 | E1 | K9 | graublau |
| 2.13 | E1 | K10 | grauschwarz |
| 2.14 | E1 | K11 | dunkelbraun |
| 2.15 | E1 | K12 | graublau |
| 2.16 | E1 | K13 | schwarzblau |
| 2.17 | E1 | K14 | blauschwarz |
| 2.18 | E1 | K15 | rehbraun |

## Patentansprüche

1. Haarfärbemittel, enthaltend Oxidationsfarbstoffvorprodukte in einem kosmetischen Träger, dadurch gekennzeichnet, daß als Oxidationsfarbstoffvorprodukt wenigstens eine Verbindung der Formel (I)

(I)

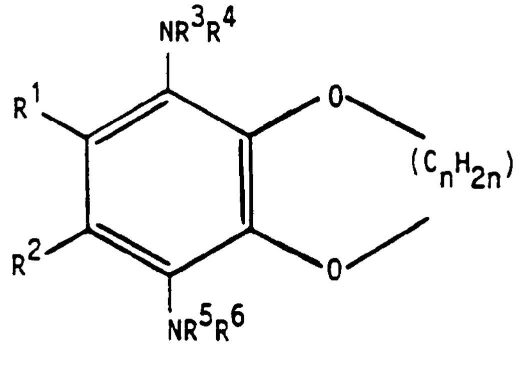

in der $R^1$ und $R^2$ unabhängig voneinander Wasserstoff, Halogenatome oder Alkylgruppen mit 1 - 4 C-Atomen und $R^3$, $R^4$, $R^5$ und $R^6$ unabhängig voneinander Wasserstoff, Alkylgruppen mit 1 - 4 C-Atomen oder

Hydroxyalkylgruppen mit 2 - 4 C-Atomen sind und n eine ganze Zahl von 2 bis 4 ist, oder deren Salz als Entwicklerkomponente enthalten ist.

2. Haarfärbemittel nach Patentanspruch 1, dadurch gekennzeichnet, daß als Oxidationsfarbstoffvorprodukt wenigstens eine Verbindung der Formel I gemäß Anspruch 1, in der $R^1$ und $R^2$ Wasserstoff, Brom oder Chlor, $R^3$, $R^4$, $R^5$ und $R^6$ Wasserstoff und $(C_nH_{2n})$ eine Ethylen- oder 1,3-Propylengruppe ist, oder deren wasserlösliches Salz enthalten ist.

3. Haarfärbemittel nach Anspruch 1, dadurch gekennzeichnet, daß als Oxidationsfarbstoffvorprodukte wenigstens eine Verbindung der Formel I sowie weitere, übliche Entwickler- und Kupplerkomponenten enthalten sind.

4. Haarfärbemittel nach Anspruch 3, dadurch gekennzeichnet, daß die weiteren Kupplerkomponenten ausgewählt sind aus der Gruppe der Aminophenole, Resorcine, Naphthole und/oder Dihydroxy-naphthaline.

5. Haarfärbemittel nach Anspruch 4, dadurch gekennzeichnet, daß die weiteren Kupplerkomponenten ausgewählt sind aus der Gruppe der Verbindungen 5-Amino-4-chlor-2-methylphenol, 3-Amino-2-chlor-6-methylphenol, 2,4-Dichlor-3-aminophenol, Resorcin, Naphthol-1 und 1,7-Dihydroxynaphthalin.

6. Haarfärbemittel nach einem der Ansprüche 3 bis 5, dadurch gekennzeichnet, daß Entwicklerkomponenten und Kupplerkomponenten in einem Molverhältnis von 1 : 0,5 bis 1 : 2 enthalten sind und der Gehalt an Oxidationshaarfärbevorprodukten 0,2 - 5 Gew.-% des Haarfärbemittels sowie der Gehalt an Verbindungen der Formel I oder deren Salzen 0,05 bis 10 Millimol pro 100 g des Haarfärbemittels beträgt.

## Claims

1. Hair-dyeing preparations containing oxidation dye precursors in a cosmetic carrier, characterized in that at least one compound corresponding to formula I

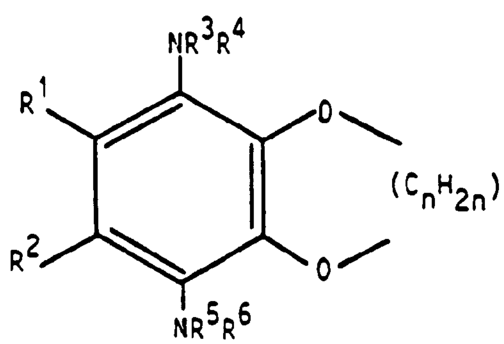

in which $R^1$ and $R^2$ independently of one another represent hydrogen, halogen atoms or $C_{1-4}$ alkyl groups and $R^3$, $R^4$, $R^5$ and $R^6$ independently of one another represent hydrogen,
$C_{1-4}$ alkyl groups or $C_{2-4}$ hydroxyalkyl groups and n is an integer of 2 to 4,
or a salt thereof is present as the developer component of the oxidation dye precursor.

2. Hair-dyeing preparations as claimed in claim 1, characterized in that at least one compound corresponding to formula I in claim 1, in which $R^1$ and $R^2$ are hydrogen, bromine or chlorine, $R^3$, $R^4$, $R^5$ and $R^6$ are hydrogen and $(C_nH_{2n})$ is an ethylene or 1,3-propylene group,
or a water-soluble salt thereof is present as the oxidation dye precursor.

3. Hair-dyeing preparations as claimed in claim 1, characterized in that at least one compound corresponding to formula I and other typical developer and coupler components are present as the oxidation dye precursors.

4. Hair-dyeing preparations as claimed in claim 3, characterized in that the other coupler components are selected from the group consisting of aminophenols, resorcinols, naphthols and/or dihydroxynaphthalenes.

5. Hair-dyeing preparations as claimed in claim 4, characterized in that the other coupler components are selected from the group consisting of the compounds 5-amino-4-chloro-2-methylphenol, 3-amino-2-chloro-6-methylphenol, 2,4-dichloro-3-aminophenol, resorcinol, 1-naphthol and 1,7-dihydroxynaphthalene.

6. Hair-dyeing preparations as claimed in any of claims 3 to 5, characterized in that the developer components and coupler components are present in a molar ratio of 1:0.5 to 1:2 and the content of oxidation hair dye precursors is from 0.2 to 5% by weight, based on the hair-dyeing preparation as a whole, while the content of compounds corresponding to formula I or salts thereof is from 0.05 to 10 millimol per 100 g of the hair-dyeing preparation.

## Revendications

1. Teintures capillaires contenant des précurseurs de colorant d'oxydation dans un véhicule cosmétique, caractérisées en ce qu'elles contiennent comme précurseur de colorant d'oxydation au moins un composé de formule I.

(I)

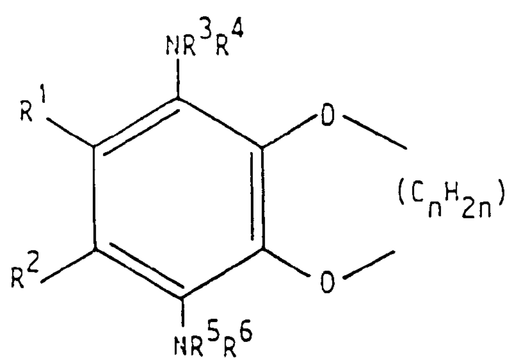

dans laquelle $R^1$ et $R^2$ représentent indépendamment l'hydrogène, des atomes d'halogène ou des groupes alkyles avec 1 à 4 atomes de C et $R^3$, $R^4$, $R^5$ et $R^6$ représentent indépendamment l'hydrogène, des groupes alkyles avec 1 - 4 atomes de C ou des groupes hydroxyalkyles avec 2 - 4 atomes de C et n est un nombre entier entre 2 et 4,
ou leurs sels, en tant que composant développeur.

2. Teintures capillaires selon la revendication 1, caractérisées en ce qu'elles comprennent comme précurseur de colorant d'oxydation au moins un composé de formule 1 selon la revendication 1, dans laquelle $R^1$ et $R^2$ sont l'hydrogène, le brome ou le clore, $R^3$, $R^4$, $R^5$ et $R^6$ sont l'hydrogène et $C_nH_{2n}$ un groupe éthylène ou 1,3-propylène ou un de ses sels solubles dans l'eau.

3. Teintures capillaires selon la revendication 1, caractérisées en ce qu'elles comprennent comme précurseurs de colorants d'oxydation au moins un composé de formule I ainsi que d'autres composants développeurs et coupleurs.

4. Teintures capillaires selon la revendication 3, caractérisées en ce qu'on choisit d'autres composants coupleurs dans le groupe des aminophénols, résorcines, naphtols et/ou dihydroxynaphtalènes.

5. Teintures capillaires selon la revendication 4, caractérisées en ce que les autres composants coupleurs sont choisis dans le groupe des composés 5-amino-4-chloro-2-méthylphénol, 3-amino-2-chloro-6-méthylphénol, 2,4-dichloro-3-aminophénol, résorcine, naphtol-1 et 1,7-dihydroxynaphtalène.

6. Teintures capillaires selon l'une des revendications 3 à 4, caractérisées en ce que les composants développeurs et composants coupleurs sont présents dans une proportion molaire de 1 : 0,5 à 1 : 2 et que la teneur en précurseurs de colorants d'oxydation représente 0,2 à 5 % en poids de la teinture ainsi que la teneur en composés de formule I ou leurs sels est de 0,05 à 10 millimoles pour 100 g de la teinture.